# EUROPÄISCHE PATENTANMELDUNG

(11) **EP 2 230 018 A1**
(43) Veröffentlichungstag der Anmeldung: **22.09.2010**
(21) Anmeldenummer: 09002722.8
(22) Anmeldetag: 26.02.2009
(51) Int. Cl.: B01L 99/00, G01N 33/487, G01N 35/00

(54) **Verbrauchselementemagazin für ein Messsystem zur Bestimmung einer Analytkonzentration**

(71) Anmelder: Roche Diagnostics GmbH, 68305 Mannheim (DE); F.HOFFMANN-LA ROCHE AG, 4070 Basel (CH)
(72) Erfinder: Hönes, Joahim, Dr., 64673 Zwingenberg (DE); Kràmer, Uwe, Dr., 68549 Ilvesheim (DE)
(74) Vertreter: Twelmeier Mommer & Partner

(57) **Zusammenfassung**

Die Erfindung betrifft ein Magazin für ein Gerät eines Messsystems zur Bestimmung einer Analytkonzentration in einer Körperflüssigkeitsprobe, wobei das Magazin (1) in mehreren Kammern (2a-2l, 3a-3l) analytische Verbrauchselemente (2, 3) enthält. Erfindungsgemäß ist vorgesehen, dass an dem Magazin (1) ein Datenträger (5) befestigt ist, in dem eine Information gespeichert ist, aus der hervorgeht, welche Magazinkammern (2g) wegen Fertigungsfehlern (4) nicht zur Entnahme eines Verbrauchselements (2, 3) vorgesehen sind. Die Erfindung betrifft ferner Gerät (10), dessen Steuereinheit einen Zugriff seiner Entnahmeeinrichtung auf vorgegebene Magazinkammern (2g) unterbindet, und ein Messsystem mit einem solchen Gerät (10) und einem Magazin (1) sowie ein Verfahren zum Steuern der Entnahmeeinrichtung eines Geräts (10).

## Beschreibung

Die Erfindung betrifft ein Magazin für ein Gerät eines Messsystems zur Bestimmung einer Analytkonzentration in einer Körperflüssigkeitsprobe. Ein derartiges Magazin, das in mehreren Kammern analytische Verbrauchselemente, beispielsweise Lanzetten oder Testelemente, enthält, ist aus der EP 0 951 939 B1 bekannt. Die Erfindung betrifft ferner ein Messsystem mit einem solchen Magazin und ein Verfahren zum Steuern einer Entnahmeeinrichtung eines solchen Geräts, mit der Verbrauchselemente aus Kammern eines eingesetzten Magazins entnommen werden können.

Magazine von Verbrauchselementen für Messsysteme zur Bestimmung von Analytkonzentrationen in menschlichen oder tierischen Körperflüssigkeitsproben müssen strenge Fertigungstoleranzen einhalten, damit die Kammern eines Magazins automatisch mit Verbrauchselementen gefüllt und von einer Entnahmeeinrichtung eines Handgeräts unbeschädigt entnommen werden können. Bereits kleine Abweichungen von einer vorgesehenen Kammergeometrie können dazu führen, dass sich ein Verbrauchselement in einer Magazinkammer verklemmt und deswegen nicht mehr entnommen werden oder die betreffende Magazinkammer gar nicht erst mit einem Verbrauchselement bestückt werden kann.

Üblicherweise werden derartige Magazine weggeworfen, nachdem die in ihnen enthaltenen Verbrauchselemente benutzt wurden. Die mit der Herstellung von Magazinen verbundenen Kosten machen deshalb einen erheblichen Teil der Gesamtkosten eines Messsystems bzw. einer Messung zur Bestimmung einer Analytkonzentration einer Körperflüssigkeitsprobe aus. Dies gilt insbesondere auch bei Magazinen mit einer relativ großen Zahl von Kammern für kleine Verbrauchselemente, da mit zunehmender Miniaturisierung die einzuhaltenden Fertigungstoleranzen strenger werden. Andererseits müssen jedoch die Kosten eines Magazins auf die Anzahl der darin enthaltenen Verbrauchselemente und damit auf die Anzahl der mit dem Magazin möglichen Messungen umgelegt werden, was tendenziell zu steigenden Gesamtkosten bei einer reduzierten Anzahl von Magazinkammern führt.

Aufgabe der vorliegenden Erfindung ist es deshalb, einen Weg aufzuzeigen, wie bei einem Messsystem zur Bestimmung einer Analytkonzentration einer Körperflüssigkeitsprobe die Kosten pro Messung gesenkt werden können.

Diese Aufgabe wird durch ein Magazin mit den im Anspruch 1 angegebenen Merkmalen, durch ein Messsystem gemäß Anspruch 7 und ein Gerät gemäß Anspruch 14 sowie ein Steuerungsverfahren gemäß Anspruch 15 gelöst. Vorteilhafte Weiterbildungen der Erfindung sind Gegenstand von Unteransprüchen.

Anstatt die mit dem Magazin verbundenen Kosten einer Messung durch einen optimalen Kompromiss zwischen den mit steigender Kammernanzahl zunehmenden Herstellungskosten eines Magazins und der mit sinkender Kammernanzahl kleiner werdenden Basis, auf welche die Herstellungskosten eines Magazins umgelegt werden können, zu suchen, geht die vorliegende Erfindung einen anderen Weg und nimmt Herstellungsfehler bei einzelnen Magazinkammern bewusst in Kauf.

Indem bei einem erfindungsgemäßen Magazin Fertigungstoleranzen nicht zwingend für alle Kammern eingehalten werden müssen, sondern die Unbrauchbarkeit einzelner Kammern in Kauf genommen wird, lassen sich Herstellungskosten in erheblichem Umfang einsparen. Der Aufwand, bei einem Magazin mit beispielsweise 20 oder mehr Kammern alle Kammern fehlerfrei herzustellen ist wesentlich größer als für ein entsprechend großes Magazin, bei dem man in Kauf nimmt, dass einige Kammern wegen Fertigungsfehlern nicht brauchbar sind.

Welche Kammern bei einem erfindungsgemäßen Magazin wegen Fertigungsfehlern nicht zur Entnahme eines Verbrauchselements vorgesehen sind, kann die Steuereinheit eines Geräts durch Auslesen eines an dem Magazin befestigten Datenträgers ermitteln. Obwohl bei einem erfindungsgemäßen Magazin unter Umständen einige Kammern nicht funktionsfähig sind, kann das Magazin dennoch ohne Beeinträchtigung des Benutzerkomforts benutzt werden.

Die wegen Fertigungsfehlern nicht zur Entnahme von Verbrauchselementen vorgesehenen Magazinkammern können leer sein, müssen es jedoch nicht. Eine besonders einfache Fertigung lässt sich in vielen Fällen nämlich dadurch erreichen, dass alle Magazinkammern mit Verbrauchselementen bestückt werden. Zwar können die Verbrauchselemente aus defekten Kammern nicht ohne Beschädigung, bzw. überhaupt nicht, entnommen werden, jedoch lässt sich die Befüllung besonders rationell gestalten, wenn alle Kammern gefüllt werden, was in der Regel möglich ist, wenn man eine Beschädigung des Verbrauchselements bei einem mehr oder weniger gewaltsamen einbringen in Kauf nimmt.

Bevorzugt enthält jede Magazinkammer höchsten ein einziges Verbrauchselement. Prinzipiell ist es aber möglich, in einer Kammer zwei Verbrauchselemente, beispielsweise eine Lanzette und einen separaten Teststreifen zu lagern.

Bevorzugt haben die Magazinkammern jeweils zwei, insbesondere gegenüberliegende, Öffnungen. Auf diese Weise lassen sich Verbrauchselemente besonders rationell entnehmen, indem ein Entnahmeelement in eine Öffnung eingeschoben und dadurch das Verbrauchselement aus einer anderen Öffnung heraus geschoben wird.

Prinzipiell genügt bei einer entsprechenden Ausgestaltung der Entnahmeeinrichtung aber eine einzige Öffnung pro Magazinkammer.

Der an einem erfindungsgemäßen Magazin befestigte Datenträger kann beispielsweise ein Barcode sein. Bevorzugt handelt es sich bei dem Datenträger jedoch um einen elektronischen Speicher, beispielsweise ein Flash-EEPROM oder ein RFID-Chip.

Die in einem erfindungsgemäßen Magazin enthaltenen Verbrauchselemente können beispielsweise Lanzetten, Testelemente oder Lanzetten mit integrierten Testelementen sein. Bevorzugt hat ein erfindungsgemäßes Magazin mindestens 20, besonders bevorzugt mindestens 40, insbesondere mindestens 50, Kammern. Bevorzugt ist bei einem erfindungsgemäßen Magazin mindestens eine Kammer wegen eines Fertigungsfehlers nicht zur Entnahme eines Verbrauchselements vorgesehen, beispielsweise zwei bis vier Kammern. Besonders bevorzugt sind beim erfindungsgemäßen Magazin bis zu 10 % der Kammern nicht zur Entnahme eines Verbrauchselements vorgesehen.

Eine vorteilhafte Weiterbildung der Erfindung sieht vor, dass die wegen Fertigungsfehlern nicht zur Entnahme eines Verbrauchselements vorgesehenen Magazinkammern ebenso wie die zur Entnahme eines Verbrauchselements vorgesehenen Magazinkammern mit Folie verschlossen sind. Auf diese Weise lässt sich die Fertigung besonders effizient gestalten. Die mit Folie versiegelten Magazinkammern, die wegen Fertigungsfehlern nicht zur Entnahme eines Verbrauchselements vorgesehenen sind, können Verbrauchselemente enthalten oder leer sein. Bevorzugt sind alle Magazinkammern mit Folie verschlossen.

Insbesondere bei Rotationsmagazinen, beispielsweise Diskus-, Trommel- Revolvermagazinen, die in dem Gerät schrittweise gedreht werden, kann es aber auch vorteilhaft sein, eine Kammer nicht mit Folie zu verschließen und nicht zu befüllen. Mit einer solchen Kammer kann in einfacher Weise eine eindeutige Nummerierung der Kammern erreicht werden, indem die leere, offene Kammer die erste Kammer ist und die übrigen Kammern von dort fortlaufend im oder gegen den Uhrzeigersinn durchgezählt werden. Auf dem Datenträger eines Magazins können beispielsweise die Nummern der wegen Fertigungsfehlern nicht zur Entnahme eines Verbrauchselements vorgesehen Kammern gespeichert sein. Möglich ist es auch, dass auf dem Datenträger alternativ oder zusätzlich die Nummern der funktionsfähigen Kammern gespeichert sind.

Zu einem erfindungsgemäßen Messsystem zur Bestimmung einer Analytkonzentration einer Körperflüssigkeitsprobe gehört neben einem Magazin auch ein Gerät, in welches das Magazin einsetzbar ist und welches eine Entnahmeeinrichtung zum Entnehmen der Verbrauchselemente aus den Magazinkammern aufweist. Ein erfindungsgemäßes Gerät ist bevorzugt ein Handgerät und hat ein Fach zum Einlegen eines Magazins, das in mehreren Kammern analytische Verbrauchselemente enthält, und eine Entnahmeeinrichtung zum Entnehmen der Verbrauchselemente aus Kammern eines eingelegten Magazins sowie eine Steuereinheit, welche einen Zugriff der Entnahmeeinrichtung auf vorgegebene Kammern unterbindet.

Bevorzugt wird ein Zugriff auf Kammern unterbunden, die wegen Fertigungsfehlern nicht zur Entnahme von Verbrauchselementen vorgesehen sind. Möglich ist es aber, dass die Steuereinheit nur einen vorgegebene Maximalanzahl von Messungen pro Magazin erlaubt und eine Entnahme überzähliger Verbrauchselemente verhindert. Dabei ist möglich, die eine Entnahme überzähliger Verbrauchselemente stets zu unterbinden oder aber von der Erfüllung vorgegebener Bedingungen abhängig zu machen. Beispielsweise können überzählige Verbrauchselemente verwendet werden, um Bedienungsfehler auszugleichen, so dass ein Benutzer trotz eines gescheiterten Probenentnahme- oder Messversuchs die für ein Magazin vorgesehene Anzahl von Messungen vornehmen kann. Möglich ist es auch, zunächst blockierte Kammern frei zuschalten, um einen Benutzer zu belohnen, beispielsweise für das regelmäßige Durchführen von Messungen.

Das Gerät eines erfindungsgemäßen Messsystems hat bevorzugt ein Lesegerät zum Auslesen des an dem Magazin befestigten Datenträgers und eine an das Lesegerät angeschlossene Steuereinheit, welche einen Zugriff der Entnahmeeinrichtung auf Kammern, die gemäß der auf dem Datenträger gespeicherten Informationen wegen Fertigungsfehlern nicht zur Entnahme eines Verbrauchselements vorgesehen sind, unterbindet. Vorteilhaft kann durch die Steuereinheit eines erfindungsgemäßen Geräts erreicht werden, dass es ein Benutzer gar nicht bemerkt, wenn bei einem eingelegten Magazin aus einzelnen Kammern kein Verbrauchselement entnommen werden kann. Die auf dem Datenträger des Magazins gespeicherte Information ermöglicht es, dass die Entnahmeeinrichtung nur auf die Magazinkammern zugreift, aus denen auch tatsächlich ein funktionsfähiges Verbrauchselement entnommen werden kann. Unbrauchbare Kammern können einfach übersprungen werden.

Im einfachsten Fall werden bei einem erfindungsgemäßen Messsystem aus einem Magazin solange Verbrauchselemente entnommen, bis eine Entnahme funktionsfähiger Verbrauchselemente nicht mehr möglich ist und das Magazin anschließend entsorgt. Bei einem Magazin mit beispielsweise 54 Kammern, das von einem Hersteller mit der Garantie vertrieben werden kann, dass dem Magazin mindestens 50 funktionsfähige Verbrauchselemente entnommen werden können, besteht folglich die Möglichkeit, dass bis zu vier Kammern wegen Funktionsfehlern nicht zur Entnahme des Verbrauchselements vorgesehen sind. Die in den Handel gebrachten Magazine würden in einem solchen Fall also 50 bis 54 funktionsfähige Verbrauchselemente enthalten. Benutzer könnten dann also hoffen, dass aus einem erworbenen Magazin mehr als nur die garantierte Mindestanzahl von Verbrauchselementen entnommen werden kann. Mehr oder weniger häufig würden solche Hoffnungen jedoch enttäuscht werden, da manche Magazine eben tatsächlich nur die garantierte Anzahl an Verbrauchselementen enthalten.

Es kann deshalb vorteilhaft sein, wenn die Steuereinheit Zugriffe der Entnahmeeinrichtung auf Kammern eines Magazins unterbindet, sobald aus dem Magazin eine vorgegebene Maximalanzahl von Verbrauchselementen, beispielsweise die garantierte Mindestanzahl, entnommen wurde. Auf diese Weise lässt sich erreichen, dass einem Magazin stets nur die garantierte Anzahl von Verbrauchselementen entnommen werden kann. Enttäuschungen von Benutzern, die Unzufriedenheiten führen könnten, lassen sich auf diese Weise vermeiden.

Die bei den Magazinen eines erfindungsgemäßen Messsystems häufig auftretende Überbefüllung, also die Tatsache, dass die in einem Magazin enthaltene Anzahl funktionsfähiger Verbrauchselemente in der Regel größer als eine garantierte Mindestanzahl ist, kann aber auch verwendet werden, um Benutzern in Sonderfällen eine größere Anzahl an Verbrauchselementen zur Verfügung zu stellen, beispielsweise wenn mit einem Verbrauchselement keine Messung vorgenommen werden konnte oder ein offensichtlich falscher Messwert erzeugt wurde.

Wenn bei einem Handgerät beispielsweise die Stechtiefe zu gering eingestellt ist, kann es vorkommen, dass ein Lanzettenstich keine oder keine ausreichend große Körperflüssigkeitsprobe liefert. Auch können beispielsweise kalte Finger dazu führen, dass durch einen Lanzettenstich ausnahmsweise keine oder keine ausreichende Menge Körperflüssigkeit gewonnen werden kann. In diesen und ähnlichen Fällen kann die Steuereinheit eines erfindungsgemäßen Messsystems einen zusätzlichen Zugriff der Entnahmeeinrichtung auf funktionsfähige Kammern des Magazins zulassen, also die Maximalzahl von Verbrauchselementeentnahmen, nach welcher ein Zugriff der Entnahmeeinrichtung auf Kammern des Magazins unterbunden wird, erhöhen, beispielsweise um die Zahl 1. Wenn das Magazin in einem solchen Fall mehr als die garantierte Mindestanzahl an Verbrauchselementen enthält, kann auf diese Weise ein Bedienungsfehler eines Benutzers ausgeglichen werden, so dass der Benutzer mit dem Magazin die vorgesehene Anzahl von Körperflüssigkeitsuntersuchungen vornehmen kann.

Bei einer derartigen Ausführungsform ist die vorgegebene Maximalanzahl für ein eingesetztes Magazin eine Variable, die nach dem Einsetzen eines neuen Magazins auf einen Standartwert, beispielsweise die garantierte Mindestanzahl an in einem Magazin enthaltenen funktionsfähigen Verbrauchselementen, zurückgesetzt wird.

Die Entnahmeeinrichtung eines erfindungsgemäßen Geräts kann insbesondere mit einem Verfahren gesteuert werden, dass die im Anspruch 15 angegebenen Merkmale aufweist.

### Weitere Einzelheiten und Vorteile der Erfindung erläutert. Es zeigen:

- Figur 1: eine schematische Darstellung eines Ausführungsbeispiels eines erfindungsgemäßen Magazins;
- Figur 2: eine schematische Darstellung eines Handgeräts, in welches das Magazin eingesetzt werden kann.

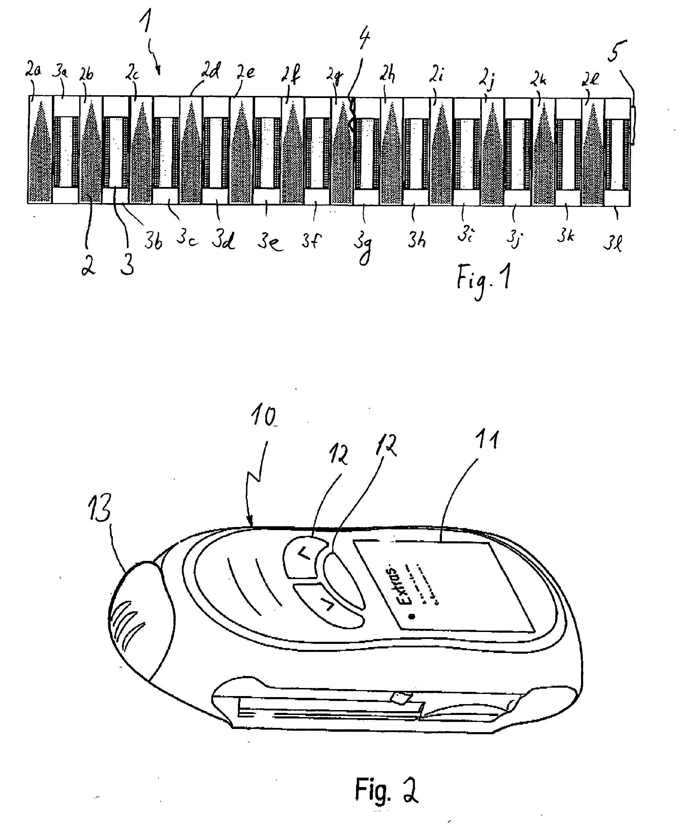

Figur 1 zeigt schematisch ein Magazin 1 für ein in Figur 2 gezeigtes Handgerät 10 eines Messsystems zur Bestimmung einer Analytkonzentration in einer Körperflüssigkeitsprobe. Das Magazin 1 enthält als Verbrauchselemente Lanzetten 2 und Testelemente 3, die jeweils einzeln in separaten Kammern 2a-21, 3a-31 des Magazins 1 angeordnet sind. Die Magazinkammern 2a-21, 3a-31 haben jeweils zwei gegenüber liegende Öffnungen, die mit Folie verschlossen sind. Durch Einschieben eines Stößels einer Entnahmeeinrichtung eines Handgeräts 10 können die Verbrauchselemente 2, 3 aus den Magazinkammern 2a-21, 3a-31 entnommen werden.

Das Magazin 1 ist ein Spritzgussteil aus Kunststoff. Durch Abnutzung der Spritzguss- , form oder fehlerhafte Spritzgussparameter kann es zu Fertigungsfehlern kommen, so dass eine oder mehrere Kammern eines solchen Magazins vorgegebene Fertigungstoleranzen nicht erfüllen und deshalb nicht brauchbar sind.

Bei dem dargestellten Ausführungsbeispiel hat eine der Magazinkammern, nämlich die Kammer 2g, einen Fertigungsfehler 4, so dass das in dieser Kammer angeordnete Verbrauchselement 2 nicht oder nur mit großen Schwierigkeiten entnommen werden kann.

An dem Magazin 1 ist ein Datenträger 5 befestigt, in dem eine Information gespeichert ist, aus der hervorgeht, welche von den Magazinkammern 2a-2I, 3a-3I wegen Fertigungsfehlern nicht zur Entnahme eines Verbrauchselements 2, 3 vorgesehen sind. Der Datenträger 5 kann beispielsweise ein Barcode oder ein magnetischer Speicher sein. Bevorzugt ist der Datenträger 5 jedoch ein elektronischer Speicher, insbesondere ein RFID-Chip. Die in dem Datenträger 5 gespeicherte Information kann beispielsweise für jede einzelne Kammer 2a-2I, 3a-3I angeben, ob diese zur Entnahme eines Verbrauchselements 2, 3 vorgesehen ist oder nicht. Prinzipiell genügt es jedoch, beispielsweise in einer Liste die Kammern anzugeben, die wegen Fertigungsfehlern nicht zur Entnahme eines Verbrauchselements 2, 3 vorgesehen sind, oder nur die Kammern anzugeben, welche vorgegebene Fertigungstoleranzen erfüllen.

Figur 2 zeigt in einer schematischen Darstellung ein Handgerät 10, in welches das in Figur 1 dargestellte Magazin 1 eingesetzt werden kann. Bei einem solchen Handgerät 10 kann es sich beispielsweise um ein Stechgerät oder ein Messgerät zur Bestimmung einer Analytkonzentration einer Körperflüssigkeitsprobe, beispielsweise der Glucose- oder Lacktatkonzentration handeln. Das dargestellte Handgerät 10 ist zugleich ein Messgerät zur Bestimmung der Glucosekonzentration und ein Stechgerät.

Das Handgerät 10 hat eine Anzeigeeinrichtung 11, beispielsweise eine Flüssigkristallanzeige und Bedienelemente in Form von Tasten 12. Eine Schutzkappe 13 des Geräts 10 verschließt eine Gehäuseöffnung, an die ein Körperteil zur Gewinnung einer Blutprobe angelegt werden kann. Die Stromversorgung des Handgeräts 10 erfolgt mittels Batterien, die in ein Batterieaufnahmefach des Geräts eingelegt werden können.

Das Handgerät 10 hat an seiner Rückseite ein nicht dargestelltes Fach für ein Magazin 1 mit Verbrauchselementen 2, 3. Beim Einsetzen eines Magazins 1 wird der an dem Magazin 1 befestigte Datenträger 5 von einem Lesegerät des Handgeräts 10 ausgelesen und die auf dem Datenträger 5 gespeicherte Information, welche Magazinkammern wegen Fertigungsfehlern nicht zur Entnahme eines Verbrauchselements 2, 3 vorgesehen sind, an eine nicht dargestellte Steuereinheit, beispielsweise ein Mikroprozessor, weitergegeben und von dieser ausgewertet.

Das Handgerät 10 hat eine nicht dargestellte Entnahmeeinrichtung zum Entnehmen der Verbrauchselemente 2, 3 aus den Magazinkammern 2a-2I, 3a-3I. Diese Entnahmeeinrichtung kann beispielsweise als ein Stößel ausgebildet sein, der zur Entnahme in eine Magazinöffnung eindringt und ein Verbrauchselement 2, 3 aus einer entgegengesetzt angeordnete Kammeröffnung herausschiebt. Die Steuereinheit unterbindet einen Zugriff der Entnahmeeinrichtung auf Kammern, die gemäß der auf dem Datenträger 5 gespeicherten Informationen wegen Fertigungsfehlern nicht zur Entnahme eines Verbrauchselements 2, 3 vorgesehen sind, beispielsweise indem das Magazin 1 um einen Schritt weiterbewegt wird, sobald sich eine wegen Fertigungsfehlern nicht zur Entnahme eines Verbrauchselements vorgesehene Magazinkammer 2g in der Entnahmeposition befindet und die nächste Kammer 2h in die Gebrauchsposition gebracht wird, bevor die Entnahmeeinrichtung zugreift.

Die Steuerungseinrichtung des dargestellten Handgeräts 10 unterbindet Zugriffe der Entnahmeeinrichtung auf verschlossene Kammern 2a-2I, 3a-3I des Magazins 1, sobald aus dem Magazin 1 eine vorgegebene Maximalanzahl von Verbrauchselementen 2, 3 entnommen wurde. Diese Maximalanzahl kann beispielsweise einer garantierten Mindestanzahl von funktionsmittelfähigen Verbrauchselementen 2, 3 eines Magazins entsprechen. Beispielsweise könnte bei dem in Figur 1 dargestellten Magazin 1 vom Hersteller garantiert sein, dass zehn funktionsfähige Lanzetten 2 entnommen werden können. Prinzipiell könnten aus dem in Figur 1 dargestellten Magazin 1 jedoch elf Lanzetten 2 entnommen werden, da nur eine einzige Magazinkammer 2g schadhaft ist.

Die Steuereinheit kann die Entnahme einer elften Lanzette unterbinden, damit aus allen vertriebenen Magazinen 1 des Messsystems stets dieselbe Anzahl von Lanzetten 2, nämlich nur die garantierte Mindestanzahl, entnommen werden kann. Die vorgegebene Maximalanzahl kann in einem elektronischen Speicher des Handgeräts 10 gespeichert sein. Dies ist ein Beispiel für einen Fall, in dem die vorgegebene Maximalanzahl von der Anzahl der Kammern, die wegen Fertigungsfehlern nicht zur Entnahme des Verbrauchselements vorgesehen sind, unabhängig ist, also beispielsweise der vom Hersteller garantierten Minimalanzahl von funktionsfähigen Verbrauchselementen entspricht. Möglich ist es aber auch, dass die vorgegebene Maximalanzahl für ein eingesetztes Magazin 1 eine Variable ist, die nach dem Einsetzen eines neuen Magazins 1 auf einen Standardwert zurückgesetzt wird. Beispielsweise kann die Maximalanzahl von der Steuereinheit um eins erhöht werden, wenn nach einer Entnahme eines Verbrauchselements 2, 3 in einer vorgegebenen Zeit keine Messung durchgeführt wird oder das Messergebnis außerhalb eines vorgegebenen Bereichs liegt. Auf diese Weise können überzählige Verbrauchselemente 2, 3 bei gescheiterten Messversuchen genutzt werden, um einem Benutzer die vorgesehene Anzahl von Messungen pro Magazin 1 zu ermöglichen.

### Bezugszeichen

- 1: Magazin
- 2: Lanzetten
- 2a - 2I: Magazinkammern
- 2g: Schadhafte Magazinkammer
- 3a - 3I: Magazinkammern
- 4: Fertigungsfehler
- 5: Datenträger
- 10: Handgerät
- 11: Anzeigeeinrichtung
- 12: Tasten
- 13: Schutzkappe

## Patentansprüche

1. Magazin für ein Gerät eines Messsystems zur Bestimmung einer Analytkonzentration in einer Körperflüssigkeitsprobe, wobei das Magazin (1) in mehreren Kammern (2a-21, 3a-31) analytische Verbrauchselemente (2, 3) enthält, **dadurch gekennzeichnet, dass** an dem Magazin (1) ein Datenträger (5) befestigt ist, in dem eine Information gespeichert ist, aus der hervorgeht, welche Magazinkammern (2g) wegen Fertigungsfehlern (4) nicht zur Entnahme eines Verbrauchselements (2, 3) vorgesehen sind.

2. Magazin nach Anspruch 1, **dadurch gekennzeichnet, dass** der Datenträger (5) ein elektronischer Speicher ist.

3. Magazin nach einem der vorstehenden Ansprüche, **dadurch gekennzeichnet, dass** der Datenträger (5) ein RFID-Chip, ist.

4. Magazin nach einem der vorstehenden Ansprüche, **dadurch gekennzeichnet, dass** die wegen Fertigungsfehlern (4) nicht zur Entnahme eines Verbrauchselements (2, 3) vorgesehenen Magazinkammern (2g) ebenso wie die zur Entnahme eines Verbrauchselements (2, 3) vorgesehenen Magazinkammern (2a-2f, 2h-21, 3a-31) mit Folie verschlossen sind.

5. Magazin nach einem der vorstehenden Ansprüche, **dadurch gekennzeichnet, dass** die Verbrauchselemente Lanzetten (2) und/oder Testelemente (3) sind.

6. Magazin nach einem der vorstehenden Ansprüche, **dadurch gekennzeichnet, dass** die Magazinkammern (2a-21, 3a-31) jeweils höchstens ein analytisches Verbrauchselement (2, 3) enthalten.

7. Messsystem zur Bestimmung einer Analytkonzentration einer Körperflüssigkeitsprobe, mit einem Magazin (1) nach einem der vorstehenden Ansprüche und einem Gerät (10), in welches das Magazin (1) einsetzbar ist und welches eine Entnahmeeinrichtung zum Entnehmen der Verbrauchselemente (2, 3) aus den Magazinkammern (2a-21, 3a-31) aufweist, **dadurch gekennzeichnet, dass** das Gerät (10) ein Lesegerät zum Auslesen des an dem Magazin (1) befestigten Datenträgers (5) und eine an das Lesegerät angeschlossene Steuereinheit aufweist, welche einen Zugriff der Entnahmeeinrichtung auf Kammern (2g), die gemäß der auf dem Datenträger (5) gespeicherten Information wegen Fertigungsfehlern (4) nicht zur Entnahme eines Verbrauchselements (2, 3) vorgesehen sind, unterbindet.

8. Messsystem nach Anspruch 7, **dadurch gekennzeichnet, dass** die Steuereinheit Zugriffe der Entnahmeeinrichtung auf Kammern (2a-21, 3a-31) eines Magazins (1) unterbindet, sobald aus dem Magazin (1) eine vorgegebene Maximalanzahl von Verbrauchselementen (2, 3) entnommen wurde.

9. Messsystem nach Ansprüche 7 oder 8, **dadurch gekennzeichnet, dass** die vorgegebene Maximalanzahl von der Anzahl der Kammern (2g), die wegen Fertigungsfehlern (4) nicht zur Entnahme eines Verbrauchselements (2, 3) vorgesehen sind, unabhängig ist.

10. Messsystem nach Anspruch 8 oder 9, **dadurch gekennzeichnet, dass** die vorgegebene Maximalanzahl in einem elektronischen Speicher des Geräts (10) gespeichert ist.

11. Messsystem nach Anspruch 10, **dadurch gekennzeichnet, dass** die vorgegebene Maximalanzahl für ein eingesetztes Magazin (1) eine Variable ist, die nach dem Einsetzen eines neuen Magazins (1) auf einen Standardwert zurückgesetzt wird.

12. Messsystem nach Anspruch 11, **dadurch gekennzeichnet, dass** die Maximalanzahl von der Steuereinheit um Eins erhöht wird, wenn nach einer Entnahme eines Verbrauchselements (2, 3) in einer vorgegebenen Zeit keine Messung durchgeführt wird oder das Messergebnis außerhalb eines vorgegebenen Bereichs liegt.

13. Messsystem nach einem der Ansprüche 8 bis 10, **dadurch gekennzeichnet, dass** die vorgegebene Maximalanzahl eine für alle Magazine (1) des Systems gleiche Konstante ist.

14. Gerät mit einem Fach zum Einlegen eines Magazins (1), das in mehreren Kammern (2a-21, 3a-31) analytische Verbrauchselemente (2, 3) enthält, und mit einer Entnahmeeinrichtung zum Entnehmen der Verbrauchselemente (2, 3) aus den Magazinkammern (2a-21, 3a-31), **dadurch gekennzeichnet, dass** das Gerät (10) eine Steuereinheit aufweist, welche einen Zugriff der Entnahmeeinrichtung auf vorgegebene Kammern (2g) unterbindet.

15. Verfahren zum Steuern einer Entnahmeeinrichtung eines Geräts (10) eines Messsystems, die zur Entnahme von analytischen Verbrauchselementen (2, 3) aus Kammern (2a-2I, 3a-3I) eines in das Gerät (10) eingesetzten Magazins (1) dient, das in dem Gerät (10) schrittweise bewegt wird, so dass die Magazinkammern (2a-2I, 3a-3I) nacheinander in eine Entnahmeposition gelangen, in welcher die Entnahmeeinrichtung auf die betreffende Kammer (2a-21, 3a-31) zu greifen und ein Verbrauchselement (2, 3) entnehmen kann, wobei
- ein Datenträger (5) ausgelesen wird, der an einem Magazin (1) befestigt ist, das in Magazinkammern (2a-21, 3a-31) analytische Verbrauchselemente (2, 3) enthält,
- eine in dem Datenträger (5) gespeicherten Information ausgewertet wird, aus der hervorgeht, welche Magazinkammern (2g) wegen Fertigungsfehlern (4) nicht zur Entnahme eines Verbrauchselements (2, 3) vorgesehen sind, und
- sobald sich eine wegen Fertigungsfehlern (4) nicht zur Entnahme eines Verbrauchselements (2, 3) vorgesehene Magazinkammer (2g) in der Entnahmeposition befindet, das Magazin (1) um einen Schritt weiterbewegt und die nächste Kammer (2h) in die Entnahmeposition gebracht wird, bevor die Entnahmeeinrichtung zugreift.
